# EUROPEAN PATENT SPECIFICATION

(11) **EP 0 611 823 B1**
(45) Date of publication and mention of the grant of the patent: **24.11.1999**
(21) Application number: 94102443.2
(22) Date of filing: 17.02.1994
(51) Int. Cl.: C12P 7/48

(54) **A process for increasing the excretion rate of citric acid during the fermentation by the fungus Aspergillus niger**
Verfahren zur Verbesserung der Ausscheidungsgeschwindigkeit von Zitronensäure bei der Aspergillus niger Fermentierung
Procédé d'amélioration du taux d'éxcrétion de l'acide citrique pendant la fermentation par Aspergillus niger

(30) Priority: 17.02.1993 SI 9300084
(43) Date of publication of application: 24.08.1994
(73) Proprietor: KEMIJSKI INSTITUT, SI-61000 Ljubljana (SI)
(72) Inventor: Legisa, Matic, Dr., SI-61000 Ljubljana (SI); Gradisnik-Grapulin, Majda, SI-61370 Logatec (SI)
(74) Representative: Strehl Schübel-Hopf & Partner

(56) References cited:
- US-A- 3 936 352
- TRANS. BR. MYOL. SOC. , vol. 68, no. 3, 1977 G.B., pages 403--406, A. CHMIEL 'Kinetics of citric acid production by pre-cultivated mycelium of Aspergillus Niger.'

## Description

The present invention relates to the field of biotechnology, specifically to a new process for increasing the production rate of citric acid. In detail the present invention is directed to increasing the production rate of citric acid during fermentation of a microorganism belonging to the genus Aspergillus, preferably *Aspergillus niger,* by means of a new fermentative mode, i.e. aerobic submerged fermentation.

Sucrose present in the medium is converted into the acid by the fungus *Aspergillus niger* during the biotechnological production of citric acid. The yield of fermentation is about 80% while only the rest of sucrose is used as energy source or transformed to biomass. Citric acid as a product is relatively cheap in comparison with other biotechnological products, however the fermentation is taking place in huge, high volume, and energy consuming fermenters. Therefore, by shortening the overall fermentation time a significant reduction in price could be achieved. It is obvious that changes in the metabolism of *Aspergillus niger* must occur if increased rates of acid production are desired.

On industrial scale citric acid is usually produced by a batch fermentation. Certain ecological conditions are prerequisite in order to force fungal metabolism to excrete citric acid. It is well known, that a high initial sucrose concentration (14-22%) as well as a high dissolved oxygen tension is important. Since it is difficult to dissolve enough oxygen in a solution containing a high percentage of sucrose, due to the increased viscosity, a compromise between both parameters must be accepted at optimising the fermentation process. In such systems the rates of acid accumulation usually do not exceed the value of 1 gram of acid formed per hour in a litre of substrate.

At first we have studied the influence of ecological factors in a high citric acid yielding medium on the metabolism of *Aspergillus niger.*

A high percentage of dissolved sucrose in the medium increases the osmotic value in solution that presents a risk for the cells to lose water. In order to survive, they must accumulate intracellularly a low molecular weight substance that should be very inert, and not disturbing the metabolism. Glycerol was found to function as an osmoregulator in *Aspergillus niger* cells in the early stage of growth (Legi 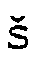 a M., Kidric J. (1989): Initiation of citric acid accumulation in the early stages of *Aspergillus niger* growth; Appl. Microbial. Biotechnol. 31, 453-457). Glycerol is formed from the intermediates of the pentose phosphate pathway that is located in the cytosol (Legi 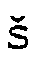 a M., Mattey M. (1986): Glycerol synthesis by *Aspergillus niger* under citric acid accumulating conditions; Enzyme Microbiol. Technol. 8, 607-609). Yet the cell membranes are not completely impermeable for glycerol since this molecule could be detected in the substrate as well (Legi 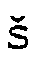 a M., Mattey M. (1986): Glycerol as an inhibitor of citric acid accumulation in *Aspergillus niger;* Enzyme Micrcobiol. Technol. 8, 258-259). Indeed, it seems very likely that glycerol can diffuse also through other cellular membranes into different compartments-including mitochondria where the enzymes of the tricarboxylic (TCA) cycle are located, It was found by in *vitro* measurements that glycerol inhibits one of the TCA cycle enzymes, namely the NADP⁺- specific isocitrate dehydrogenase. Decreased metabolic flow through this point of the TCA cycle actually results in increased levels of the citrate (Legi 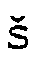 a M., Kidric J. (1989): Initiation of citric acid accumulation in the early stages of *Aspergillus niger* growth; Appl. Microbiol. Biotechnol. 31, 453-457.), due to the equilibrium constant of cis-aconitase that favours the formation of citrate (Randle P.J., Denton R.M., England P.J. (1967): Metabolic roles of citrate; In Goodwin (Ed) 27th Symp. Biochem. Soc., p. 123). Therefore, it could be concluded that a higher mitochondrial level of glycerol would result in more restricted metabolic flow through the TCA cycle and consequently in a higher intracellular level of citrate. Glycerol acts as an osmoregulator only in the early stages of growth and is dissimilated later. It seems to be only the inducer of a metabolic procedure leading to citric acid excretion. When glycerol disappears the block in the TCA cycle is believed to be maintained by increased levels of citrate itself, since it is known that this molecule is a potent inhibitor of mitochondrial NADP⁺-specific isocitrate dehydrogenase as well (Mattey M., Bowes I. (1978): Citrate regulation of NADP⁺- specific isocitrate dehydrogenase of *Aspergillus niger;* Biochem. Soc. Trans. 6, 1224-1226). Thus, the desired diminishing of the metabolic flow through the TCA cycle could be achieved by inducing more glycerol to enter the mitochondrial compartment. It could be accomplished by growing the cells in a medium with a higher initial sucrose concentration (25-30%). Under such conditions, more glycerol is synthesised intracellularly as a result of a higher osmotic value, and due to the increased concentration gradient glycerol easily passes two mitochondrial membranes and enters the matrix.

At concentrations higher than 35% of sucrose even more glycerol could be detected intracellularly but it seems that such an amount of glycerol inhibits other enzymes too, and the effect on the production of citric acid is negative.

Citric acid originally accumulates only intracellularly when the pH value is close to neutral. In such circumstances the molecule of citric acid dissociates and releases one or two protons that could significantly change the pH in the cells and influence the metabolism (Legi 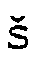 a M., Kidric J. (1989): Initiation of citric acid accumulation in the early stages of *Aspergillus niger* growth; Appl. Microbiol. Biotechnol. 31, 453-457.). It is important to realise that citric acid becomes actually toxic and must be transported out of the cells. However, a lot of energy in the form of ATP must be available to conduct the transport that can be achieved only in the presence of oxygen. It is well known that citric acid production increases simultaneously with increased dissolved oxygen tension (Kubicek C.P., Zehentgruber O., Housam El-Kalak, Rohr M. (1980): Regulation of citric acid production by oxygen: Effect of dissolved oxygen tension on adenylate levels and respiration in *Aspergillus niger;* Eur. J. Appl. Microbiol. Biotechnol. 9, 101-115). Since it is very difficult to dissolve enough oxygen in a medium with a high osmotic value, i.e. 30% sucrose solution, it was diluted. Such a procedure can be applied after 30 to 40 hours of fermentation when the block in the TCA cycle is already present. Dilutions to two third to one third of the initial sucrose concentrations yield good results although the best results were achieved by dilution of the medium by half.

Dilution is most effective if it is done instantly. When the substrate is diluted slowly, i.e within several hours, the yields obtainable have been found to be not as high. It will, however, be appreciated that depending on the reaction conditions, the fermenters employed etc. the person skilled in the art will select the appropriate dilution time. The significance of this effect shows that dilution itself presents a certain shock to the cells, and they might respond by a change in the level of intracellular cyclic AMP. According to our observation no 6-phosphofructo-1-kinase activity could be detected during the germination of spores in spite of the presence of the enzyme. The activation results later by means of phosphorylation of the protein molecule that is catalysed by cAMP-dependent protein kinase. Cyclic AMP has therefore a significant role in the activation of 6-phosphofructo-l-kinase, a limiting enzyme of the glycolytic pathway. It is important to bear in mind that the metabolic flux *via* glycolysis determines the rate of citric acid formation.

During the germination of *Aspergillus niger* cells in a medium with higher concentration of sucrose a more pronounced metabolic block in the TCA cycle is formed which significantly diminishes further citrate degradation. By diluting the substrate, oxygen is easily dissolved in the medium and enough ATP can be formed to match the energy demand of the active transport. Sudden dilution might increase the specific activity of 6-phosphofructo-1-kinase and consequently increases the metabolic flow via glycolysis. By germinating the spores in a substrate with higher sucrose concentration and by relatively sudden dilution, we succeeded in doubling the rate of citric acid production while the overall fermentation time was significantly shortened.

In accordance with the process of the present invention resulting in ten years of basic research on the metabolism of the fungus *Aspergillus niger,* we have surprisingly found that it is possible to increase the rate of citric acid excretion during the fermentation by the fungus *Aspergillus niger.* A different fermentative mode, i.e. aerobic submerged fermentation is preferably applied, instead of the hitherto used standard batch fermentation though the latter may also be used. In accordance with the process of the present invention the initial period of the fermentation is run in a medium with increased concentrations of sucrose compared to conventional media used for growing Aspergillus. Media for growing Aspergillus are well known in the art and are described e.g. in Ullmann Enzyclopädie der technischen Chemie, 1979, vol. 9, pp. 625-635. It will furthermore be appreciated that the composition of the specific medium to be used may vary depending on the given circumstances, the fungus utilized and/or the growing conditions. The selection of the appropriate composition of the medium is within the skill of the person skilled in the art and may be determined by simple experimentation. Within 30 to 40 hours of fermentation the substrate is preferably diluted by about half and the dissolved oxygen tension is kept high. Such a fermentative procedure induces a higher rate of citric acid excretion (acid formation), and thus considerably shortens the time of fermentation.

The process of the present invention for increasing the production rate of citric acid during the fermentation by the fungus Aspergillus, preferably *Aspergillus niger,* is characterised in that the fungus is grown in presence of air, preferably by means of aeration with 1 litre of air per 1 litre of substrate per minute, using the substrate with increased initial sucrose concentration compared to conventional media, containing 20 to 40%, preferably 30 to 40 % of sucrose, at a low starting pH between 2 and 3, preferably 2.5, and subsequently to the initiation of acid excretion, preferably within 30 to 40 hours of fermentation, the fermentation broth is diluted, preferably instantly to 1/3 to 2/3, preferably to 40 to 60%, most preferred to 50% of the initial sucrose concentration. The dilution is preferably effected by means of sterile deionised water having a pH between 1.5 and 2.5, preferably 2.0, while afterwards the fermentation and the isolation of the acid proceed in a conventional manner.

In a prefered embodiment the initial substrate contains about 300 grams of sucrose per litre. The diluted substrate then contains preferably about 150 grams of sucrose per litre. The substrate contains optionally salts, which are usual additives at citric acid fermentation, preferably NH₄NO₃, KH₂PO₄ and MgSO₄ The process of the present invention is illustrated by the following Examples, which are not be limiting its scope.

### Example 1

The fungus *Aspergillus niger* was grown in a high citric acid yielding medium (Legi 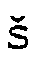 a M., Cimerman A., Sterle M. (1981): Germination of *Aspergillus niger* in a high citric acid yielding medium, FEMS Microbiol. Letts., 11, 149-152).

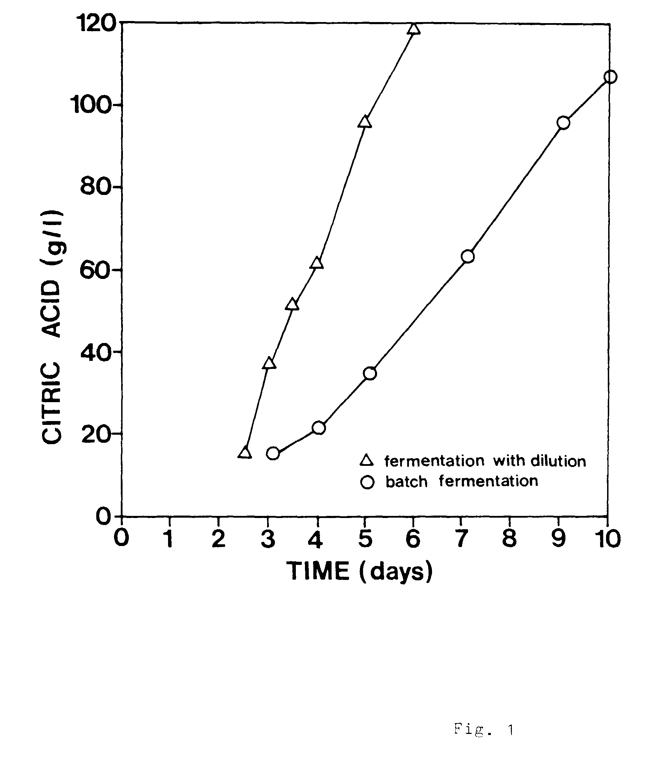

Using a batch fermentation the substrate containing 15% sucrose was inoculated by the spores and citric acid accumulation was followed. The rate of production was 0.63 grams of acid per litre of substrate per hour. Using the dilution fermentation the substrate containing 30% sucrose was inoculated with spores while the salt concentration was the same as that in batch fermentation. After 35 hours of fermentation an equal volume of deionised water (pH=2.0) was instantly poured into the fermentation broth. This procedure increased the rate of acid formation, and up to 1.6 grams of citric acid accumulated per 1 litre of substrate per hour. Air was introduced in the medium throughout the fermentation at rate of 1 litre of air per 1 litre of medium per minute. It means that after the dilution the amount of introduced air was increased due to the volume increase.
(The Diagram of Figure 1 shows a comparison of citric acid accumulation between batch fermentation and the fermentation with dilution as proposed by the present invention).

### Example 2

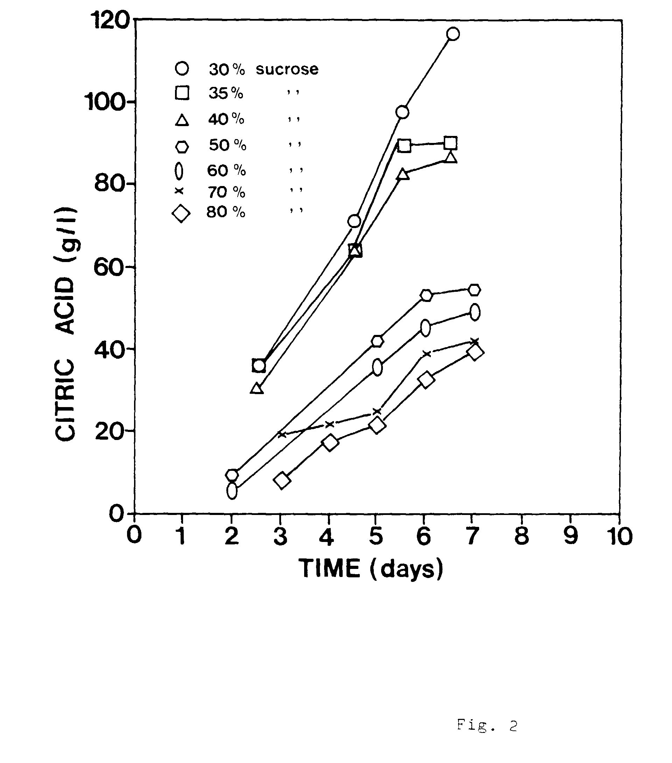

By comparing different initial sucrose concentrations in the medium for the germination of spores, prior to the dilution of the substrate to 15% sucrose concentration, it was found that the best results in acid accumulation were obtained at 30 to 40% initial sucrose concentration. Higher initial concentrations had negative effect on acid accumulation.
(The Diagram of Figure 2 shows citric acid yields at fermentations started with different sucrose concentrations).

### Example 3

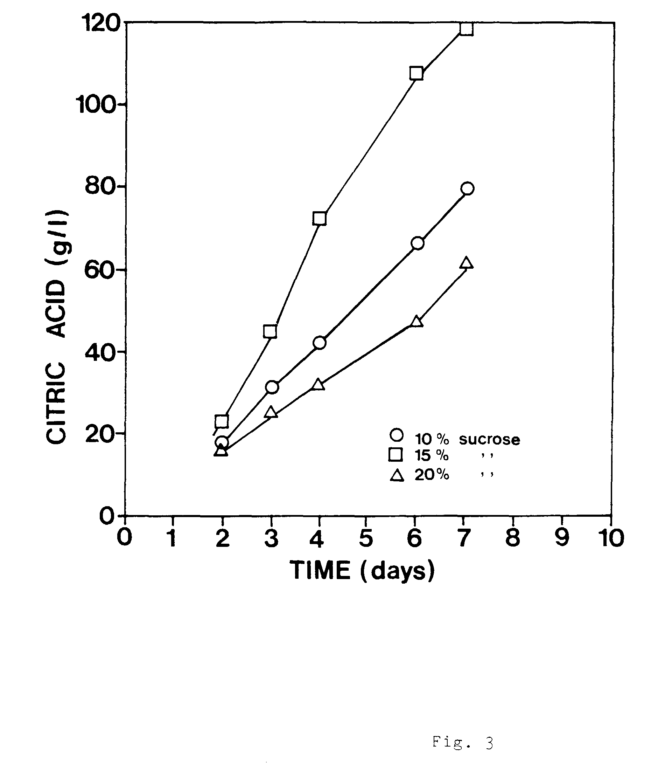

The best results were achieved if the substrate (30% sucrose concentration) was diluted by half to about 15% of sucrose concentration. However at higher or lower final concentrations also good results were obtainable.
(The Diagram of Figure 3 shows a comparison of citric acid yields at fermentations with different concentrations of sucrose after the dilution).

### Example 4

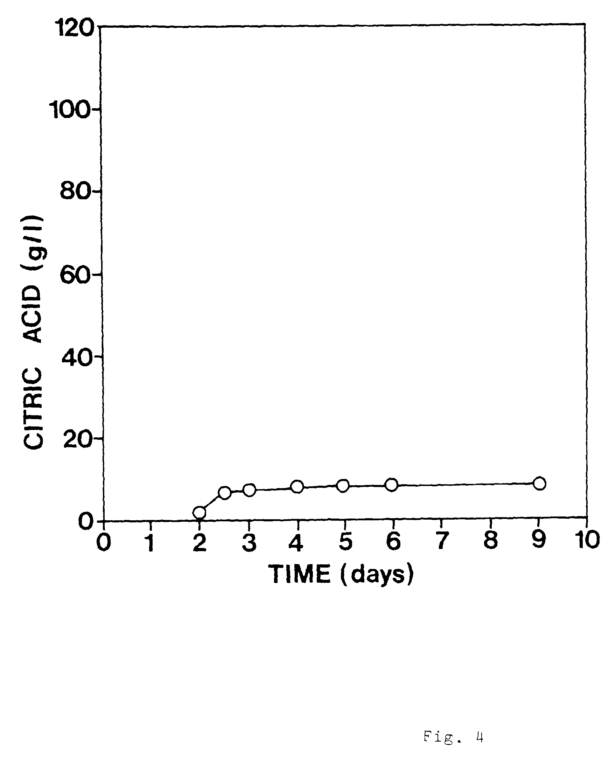

The dilution had to be relatively instant. If deionised water was added slowly, the metabolism leading to citric acid accumulation was disturbed.
(The Diagram of Figure 4 shows citric acid yields at the fermentation where the medium was gradually diluted within 10 hours).

### Example 5

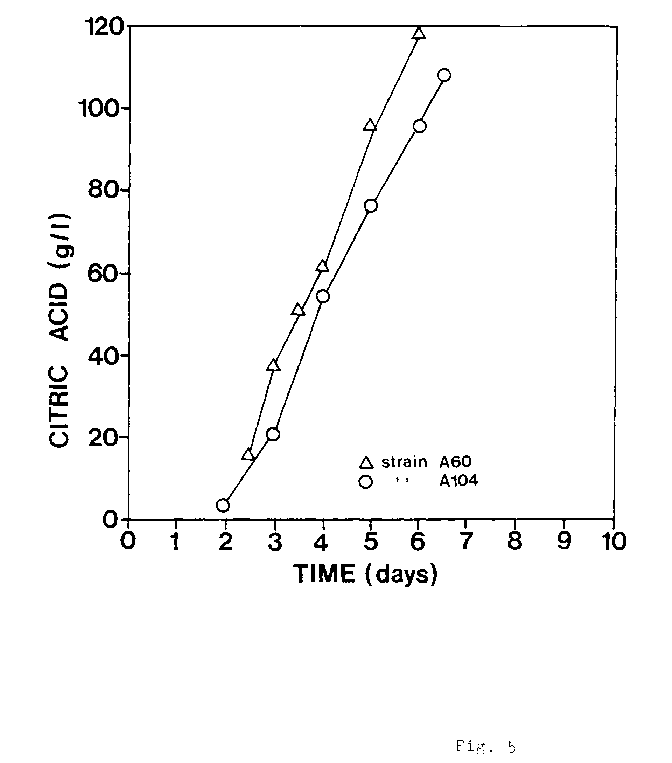

Using the dilution method in accordance with the present invention, higher rates of acid accumulation could be achieved by different strains of *Aspergillus niger,* i.e. A₆₀ (MZKIBK, Ljubljana, Slovenia) and A₁₀₄ (MZKIBK, Ljubljana, Slovenia).
(The Diagram of Figure 5 shows a comparison of citric acid yields during the fermentation after the dilution between two different *Aspergillus niger* strains).

## Claims

1. A process for the production of citric acid by fermentation characterised in that subsequent to the initiation of acid secretion by a microorganism belongig to the genus Aspergillus the fermentation broth is diluted to 2/3 to 1/3 of the initial sucrose concentration.

2. A process according to Claim 1, wherein the fermentation broth is diluted to 40 to 60 %, preferably 50 % of the initial sucrose concentration.

3. A process according to Claim 1 or Claim 2, characterised in that the microorganism used is Aspergillus niger.

4. A process according to Claim 1, 2 or 3, characterised in that the microorganism is grown in the presence of air.

5. A process according to Claim 4, characterised in that aeration is effected at a rate of 1 liter of air per 1 liter of broth per minute.

6. A process according to any of the preceding Claims, characterised in that a fermentation broth with increased initial sucrose concentration compared with a conventional fermentation medium is used for the production of citric acid.

7. A process according to Claim 6, characterised in that the initial sucrose concentration of the fermentation broth is in the range of from 20 - 40%.

8. A process according to Claim 6, characterised in that the initial sucrose concentration of the fermentation broth is in the range of from 30 - 40%.

9. A process according to any of the preceding Claims, characterised in that the fermentation broth has a starting pH in the range of from 2 to 3.

10. A process according to any of the preceding Claims, characterised in that the fermentation broth is diluted after 30 to 40 hours of fermentation.

11. A process according to any of the preceding Claims, characterised in that the fermentation broth is diluted by means of deionised water.

12. A process according to Claim 11, characterised in that the pH of the deionised water is in the range of from 1.5 to 2.5.

13. A process according to any of the preceding Claims, characterised in that the fermentation broth comprises usual additives of citric acid fermentation.

14. A process according to Claim 13, characterised in that the additives are NH₄NO₃, KH₂PO₄ and/or MgSO₄.

## Patentansprüche

1. Verfahren zur Herstellung von Zitronensäure durch Fermentation, dadurch gekennzeichnet, daß nach dem Beginn der Säuresekretion durch einen Mikroorganismus, der zur Gattung Aspergillus gehört, die Fermentationsbrühe auf 2/3 bis 1/3 der anfänglichen Saccharosekonzentration verdünnt wird.

2. Verfahren gemäß Anspruch 1, wobei die Fermentationsbrühe auf 40 bis 60%, vorzugsweise 50% der anfänglichen Saccharosekonzentration verdünnt wird.

3. Verfahren gemäß Anspruch 1 oder 2, dadurch gekennzeichnet, daß der eingesetzte Mikroorganismus Aspergillus niger ist.

4. Verfahren gemäß Anspruch 1, 2 oder 3, dadurch gekennzeichnet, daß der Mikroorganismus in Anwesenheit von Luft gezüchtet wird.

5. Verfahren gemäß Anspruch 4, dadurch gekennzeichnet, daß die Belüftung bei einer Rate von 1 l Luft pro 1 l Brühe pro Minute bewirkt wird.

6. Verfahren gemäß einem der vorstehenden Ansprüche, dadurch gekennzeichnet, daß eine Fermentationsbrühe mit im Vergleich zu einem herkömmlichen Fermentationsmedium erhöhter anfänglicher Saccharosekonzentration für die Herstellung von Zitronensäure eingesetzt wird.

7. Verfahren gemäß Anspruch 6, dadurch gekennzeichnet, daß die anfängliche Saccharosekonzentration der Fermentationsbrühe im Bereich von 20 bis 40% liegt.

8. Verfahren gemäß Anspruch 6, dadurch gekennzeichnet, daß die anfängliche Saccharosekonzentration der Fermentationsbrühe im Bereich von 30 bis 40% liegt.

9. Verfahren gemäß einem der vorstehenden Ansprüche, dadurch gekennzeichnet, daß die Fermentationsbrühe einen anfänglichen pH im Bereich von 2 bis 3 hat.

10. Verfahren gemäß einem der vorstehenden Ansprüche, dadurch gekennzeichnet, daß die Fermentationsbrühe nach 30 bis 40 Stunden der Fermentation verdünnt wird.

11. Verfahren gemäß einem der vorstehenden Ansprüche, dadurch gekennzeichnet, daß die Fermentationsbrühe mit entionisiertem Wasser verdünnt wird.

12. Verfahren gemäß Anspruch 11, dadurch gekennzeichnet, daß der pH des entionisierten Wassers im Bereich von 1,5 bis 2,5 ist.

13. Verfahren gemäß einem der vorstehenden Ansprüche, dadurch gekennzeichnet, daß die Fermentationsbrühe übliche Zusätze der Zitronensäurefermentation enthält.

14. Verfahren gemäß Anspruch 13, dadurch gekennzeichnet, daß die Zusätze NH₄NO₃, KH₂PO₄ und/oder MgSO₄ sind.

## Revendications

1. Procédé de production d'acide citrique par fermentation, caractérisé en ce que, après le début de la sécrétion d'acide par un micro-organisme appartenant au genre *Aspergillus,* on dilue le bouillon de fermentation à 2/3 à 1/3 de la concentration initiale de sucrose.

2. Procédé selon la revendication 1, dans lequel on dilue le bouillon de fermentation à 40 à 60 %, de préférence à 50 % de la concentration initiale de sucrose.

3. Procédé selon la revendication 1 ou la revendication 2, caractérisé en ce que le micro-organisme utilisé est *Aspergillus niger.*

4. Procédé selon la revendication 1, 2 ou 3, caractérisé en ce que l'on cultive le micro-organisme en présence d'air.

5. Procédé selon la revendication 4, caractérisé en ce que l'on effectue l'aération à un débit de 1 litre d'air par litre de bouillon et par minute.

6. Procédé selon l'une quelconque des revendications précédentes, caractérisé en ce que l'on utilise pour la production d'acide citrique un bouillon de fermentation ayant une concentration initiale de sucrose augmentée par comparaison à un milieu de fermentation classique.

7. Procédé selon la revendication 6, caractérisé en ce que la concentration initiale de sucrose du bouillon de fermentation est comprise entre 20 et 40 %.

8. Procédé selon la revendication 6, caractérisé en ce que la concentration initiale de sucrose du bouillon de fermentation est comprise entre 30 et 40 %.

9. Procédé selon l'une quelconque des revendications précédentes, caractérisé en ce que le bouillon de fermentation a un pH de départ compris entre 2 et 3.

10. Procédé selon l'une quelconque des revendications précédentes, caractérisé en ce que l'on dilue le bouillon de fermentation au bout de 30 à 40 heures de fermentation.

11. Procédé selon l'une quelconque des revendications précédentes, caractérisé en ce que l'on dilue le bouillon de fermentation à l'aide d'eau désionisée.

12. Procédé selon la revendication 11, caractérisé en ce que le pH de l'eau désionisée est compris entre 1,5 et 2,5.

13. Procédé selon l'une quelconque des revendications précédentes, caractérisé en ce que le bouillon de fermentation comprend des additifs courants de la fermentation d'acide citrique.

14. Procédé selon la revendication 13, caractérisé en ce que les additifs sont NH₄NO₃, KH₂PO₄ et/ou MgSO₄.
